# EUROPEAN PATENT APPLICATION

(11) **EP 4 265 244 A1**
(43) Date of publication of application: **25.10.2023**
(21) Application number: 23169176.7
(22) Date of filing: 21.04.2023
(51) Int. Cl.: A61K 31/164, A61K 31/4045, A61P 25/28

(54) **N-PALMITOYLETHANOLAMIDE AND MELATONIN FOR USE IN THE TREATMENT OF AUTISM SPECTRUM DISORDER AND OTHER NEUROBEHAVIORAL DISORDERS SIMILARLY ACCOMPANIED BY RESTLESSNESS, IRRITABILITY, SLEEP DISORDERS, AND POTENTIALLY STEREOTYPIES**

(30) Priority: 22.04.2022 IT 202200007952
(71) Applicant: EPITECH GROUP S.p.A., 20144 Milano (MI) (IT)
(72) Inventor: DELLA VALLE, Raffaella, I-20144 MILANO (IT); DELLA VALLE, Maria Federica, I-20144 MILANO (IT); MARCOLONGO, Gabriele, I-20144 MILANO (IT); GOMIERO, Chiara, I-20144 MILANO (IT); CUZZOCREA, Salvatore, I-20144 MILANO (IT); CALIGNANO, Antonio, I-20144 MILANO (IT); CRISTIANO, Claudia, I-20144 MILANO (IT)
(74) Representative: Long, Giorgio

(57) **Abstract**

The present invention relates to the use of N-palmitoylethanolamide (PEA) in association with melatonin or natural precursors thereof in the treatment of autism spectrum disorder (ASD) and other neurobehavioral disorders similarly accompanied by restlessness, irritability, sleep disorders, and potentially stereotypies.

In particular, the present invention relates to the use of PEA in association with melatonin in the treatment of ASD, wherein the palmitoylethanolamide is administered in association with melatonin or tryptophan (TRP) or 5-hydroxytriptophan (5H-TRP), wherein said administration is separate, combined or simultaneous.

## Description

### Technical field of the invention

The present invention relates to the use of N-palmitoylethanolamide (PEA) in association with melatonin or natural precursors thereof in the treatment of autism spectrum disorder (ASD) and other neurobehavioral disorders similarly accompanied by restlessness, irritability, sleep disorders, and potentially stereotypies.

### Background art

ASD is a multifactorial-etiology neurodevelopmental disorder occurring through the impairment of social interaction, verbal and non-verbal communication, activities, and interest (definition from the *American Psychiatric Association).* The symptomatology begins within the first three years of life following a specific event and early diagnosis is very important to be able to adequately intervene on the various compromised areas.

Stereotypies are one of the main diagnostic features of ASD and can comprise self-stimulatory and selfharming behaviors, excitement, stimulation, stress, anxiety, boredom, fatigue, and social isolation.

Other neurobehavioral disorders associated with the autism spectrum are restlessness, irritability, and sleep disorders.

Although the cause is not known, the appearance of ASD is also due to an imbalance between inhibitory and excitatory synapses, as well as neuroinflammation responsible for the activation and pathological proliferation of non-neuronal cells which, by releasing cytokines (IL-1α, IL-1β, IL-6 and TNF-α) and proinflammatory chemokines (MCP-1 and RANTES) in the brain and cerebrospinal fluid of autistic patients, exacerbate the neuroinflammatory process.

Sleep is one of the essential needs for the development and maturation of the brain. Children with ASD and disturbed sleep have an exacerbation of developmental delays in cognitive functions such as attention, memory consolidation, mood regulation, and social interaction.

Furthermore, the involvement of altered myelination has recently been highlighted in both experimental models of autism and in human patients with ASD. It is known that the administration of melatonin, a hormone produced naturally by the pineal gland (or epiphysis), is an aid in regulating circadian rhythms and reducing sleep disturbances, stereotyped repetitive behaviors and attention deficit.

The endogenous molecule PEA is known to play an important role in the natural modulation mechanism of neuroinflammation. In pre-clinical and clinical settings, the administration of PEA, especially if in an ultra-micronized form (um-PEA), is capable of determining neuroinflammatory normalization activity; in particular, it has been demonstrated that um-PEA is capable of significantly counteract the general neuroinflammatory status of mice with a simil-autistic phenotype, reducing the expression of the proinflammatory hippocampal and serum cytokines IL-6, IL-1β and TNF-α, as well as modulating the altered behavioral status. Clinically, the 3-month treatment of autistic children with um-PEA 600 mg/day improves aggressiveness, cognitive and behavioral skills, and communication without adverse effects.

Therefore, there is a need to provide ASD therapy, and in particular for neurobehavioral disorders similarly accompanied by restlessness, irritability, sleep disorders, which is effective, non-invasive and safe and which, if possible, does not require the administration of high doses of active substances. In fact, taking into account that such therapy would be primarily dedicated to a child population, repeated administration and/or in large dosage forms (for example, large tablets for oral administration of high doses of active ingredients) would be difficult to accept by the patient.

### Summary of the invention

The present invention derives from the surprising discovery that PEA, preferably when used in ultra-micronized form, when administered in association with melatonin, exhibits a synergistically relevant effect in improving the behavioral parameters of autistic subjects, in particular the parameters associated with restlessness, irritability, sleep disorders and stereotypies.

Therefore, the present invention relates to PEA for use in the treatment of ASD, where PEA is administered in association with melatonin, where said administration is separate, combined, or simultaneous.

The invention further relates to a composition containing PEA and melatonin, in particular when usable in the treatment of ASD.

These and further objects, as outlined in the appended claims, will be described in the following description. The text of the claims should be considered included in the description in order to assess the description sufficiency.

Further features and advantages of the invention will become apparent from the following description of preferred embodiments, given by way of non-limiting examples.

### Brief description of the drawings

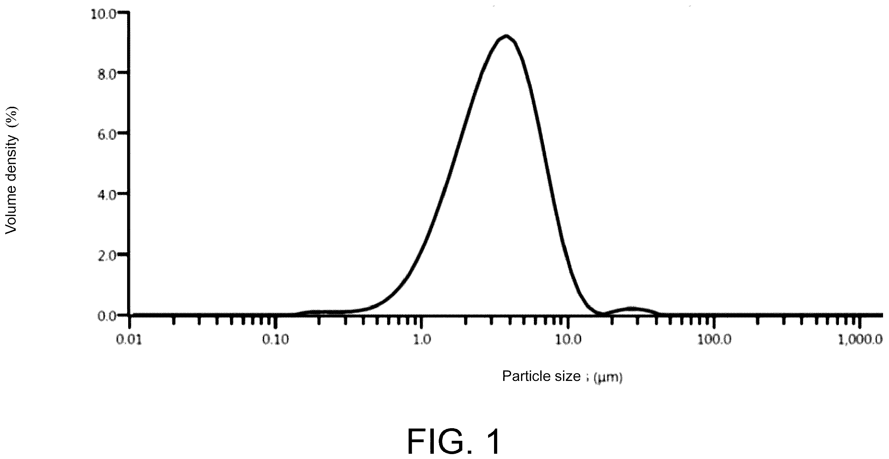

Figure 1 shows a graph of particle size distribution of palmitoylethanolamide in an ultra-micronized form (um-PEA) ;

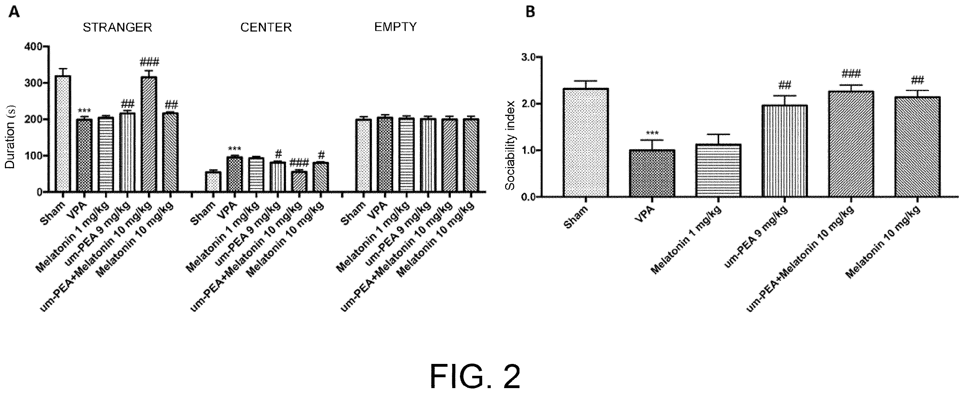

Figure 2 shows a graph of the sociability index (ratio of the time spent by the mouse to explore the area without and with the new companion) following the treatment according to the invention compared with the separate active substances (***p < 0.001 vs. Sham; #p < 0.05 vs. VPA; ##p < 0.01 vs. VPA; ###p < 0.001 vs. VPA);

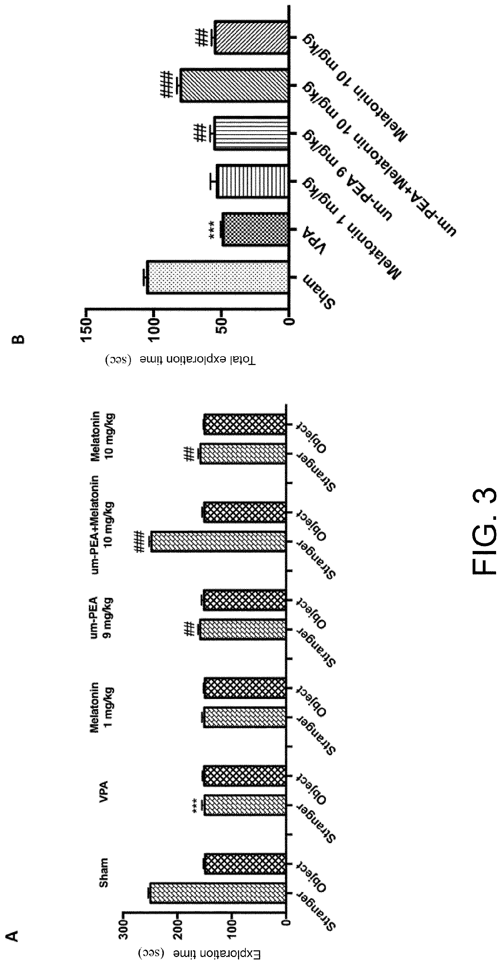

Figure 3 shows graphs of: A) time for exploring a new object with respect to a familiar object (indicated as "stranger" and "object" in histogram A below, respectively) by means of the treatment according to the invention compared with um-PEA alone (9 mg/kg) and melatonin alone (1 mg/kg and 10 mg/kg); B) total exploration time of the objects by means of the treatment according to the invention compared with um-PEA alone (9 mg/kg) and melatonin alone (1 mg/kg and 10 mg/kg) (***p < 0.001 vs. Sham; ##p < 0.01 vs. VPA; ###p < 0.001 vs. VPA);

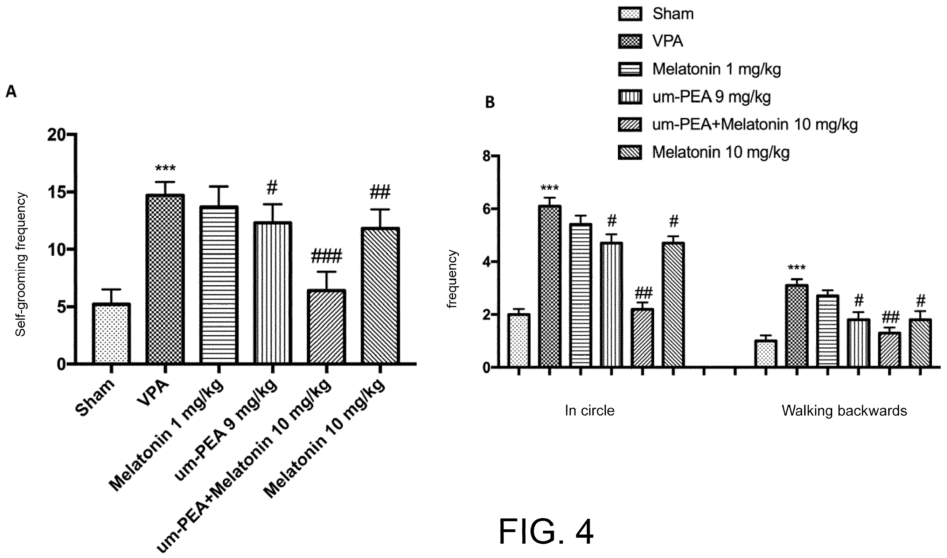

Figure 4 shows graphs of stereotyped repetitive behavior as a response to the treatment according to the invention, compared with um-PEA alone (9 mg/kg) and melatonin alone (1 mg/kg and 10 mg/kg); the stereotyped repetitive behavior is measured by using the test of self-grooming (histogram A) and the analysis of unnatural repetitive movements such as circling and walking backwards (histogram B) (***p < 0.001 vs. Sham; #p < 0.05 vs. VPA; ##p < 0.01 vs. VPA; ###p < 0.001 vs. VPA) ;

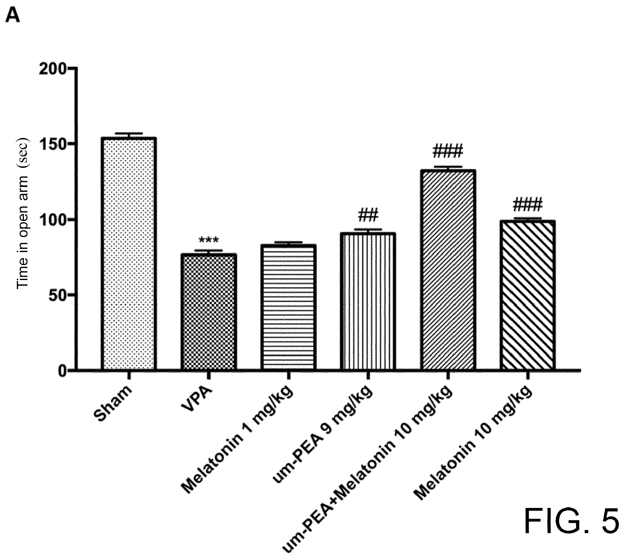

Figure 5 shows a graph of increased time spent in the open arm of the elevated plus maze (EPM) by means of the treatment according to the invention compared with um-PEA alone (9 mg/kg) and melatonin alone (1 mg/kg and 10 mg/kg) (***p < 0.001 vs. Sham; ##p < 0.01 vs. VPA; ###p < 0.001 vs. VPA);

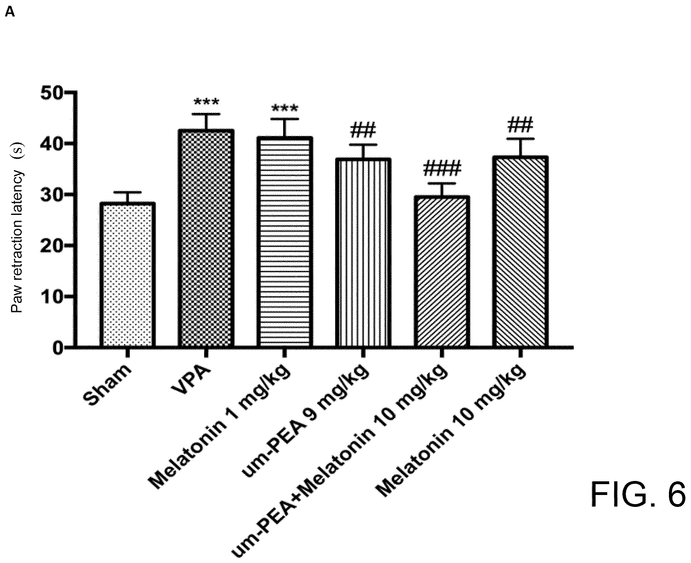

Figure 6 shows a graph regarding the pain sensitivity (hot plate) wherein the increase of latency induced by the VPA is counteracted by the treatment according to the invention more than with respect to the separate active substances (***p < 0.001 vs. Sham; ##p < 0.01 vs. VPA; ###p < 0.001 vs. VPA);

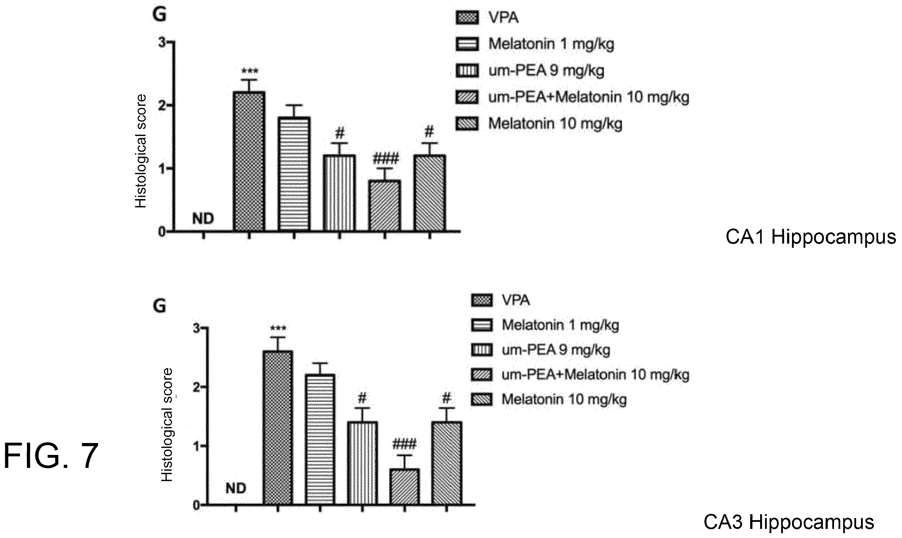

Figure 7 shows a graph of reduction of histological score of neurodegeneration of pyramidal cells in the CA1 and CA3 regions of the hippocampus by means of the treatment according to the invention compared with um-PEA alone (9 mg/kg) and melatonin alone (1 mg/kg and 10 mg/kg) (***p < 0.001 vs. Sham; #p < 0.05 vs. VPA; ###p < 0.001 vs. VPA);

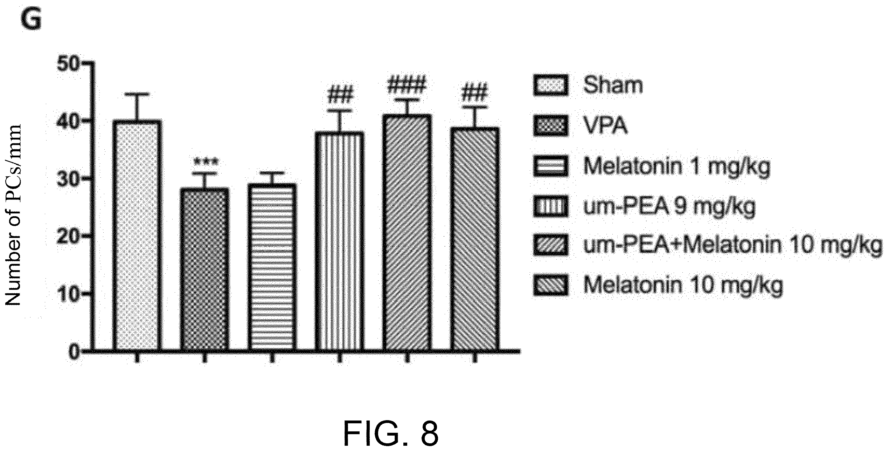

Figure 8 shows a graph of increase of the density of Purkinje cells in the cerebellum by means of the treatment according to the invention compared with um-PEA alone (9 mg/kg) and melatonin alone (1 mg/kg and 10 mg/kg) (***p < 0.001 vs. Sham; ##p < 0.01 vs. VPA; ###p < 0.001 vs. VPA);

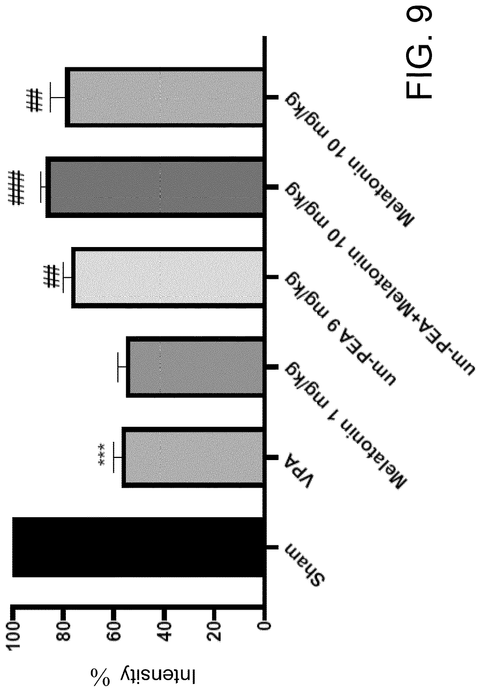

Figure 9 shows a graph of the re-myelination of the myelin sheath by means of the treatment according to the invention compared with um-PEA alone (9 mg/kg) and melatonin alone (1 mg/kg and 10 mg/kg) (***p < 0.001 vs. Sham; ##p < 0.01 vs. VPA; ###p < 0.001 vs. VPA);

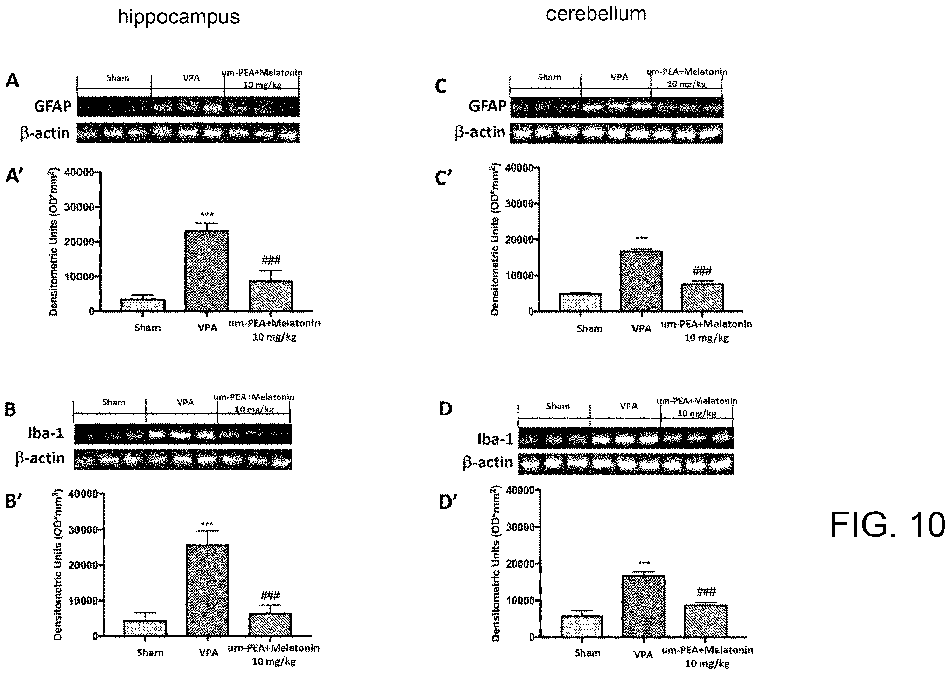

Figure 10 shows graphs related to the reduction by the composition of the invention of VPA-induced astrogliosis and microgliosis at the hippocampus and cerebellum level, as demonstrated by the lower expression, respectively, of the specific markers GFAP and Iba-1 (***p < 0.001 vs. Sham; ###p < 0.001 vs. VPA);

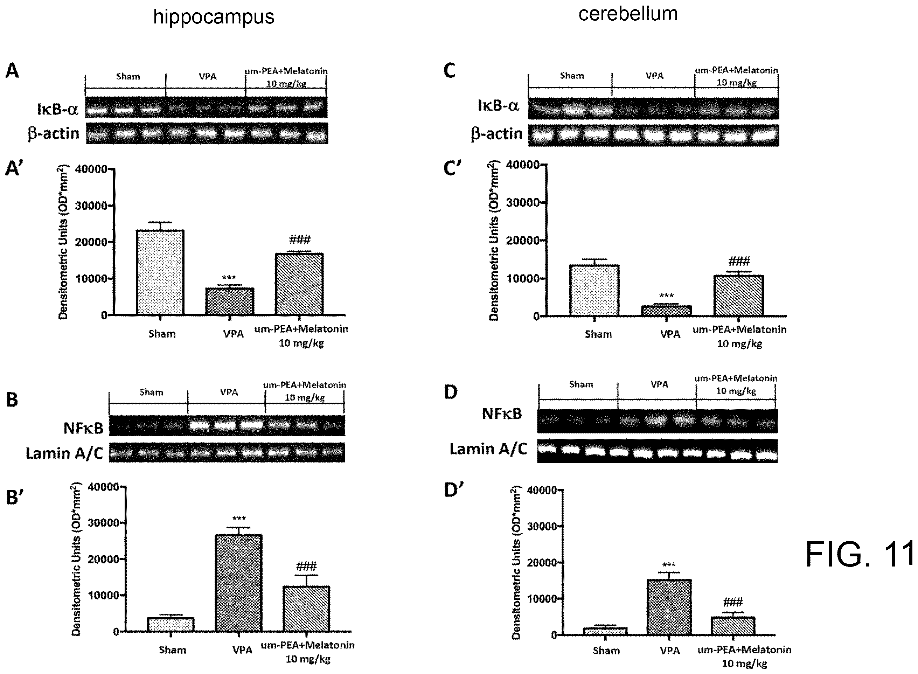

Figure 11 shows graphs related to the ability of the inventive composition to counteract the effects of VPA on proinflammatory nuclear factor (NF-kB) and on the "inhibitor" thereof (IkB-α) at the cerebellum and hippocampus level (***p < 0.001 vs. Sham; ###p < 0.001 vs. VPA);

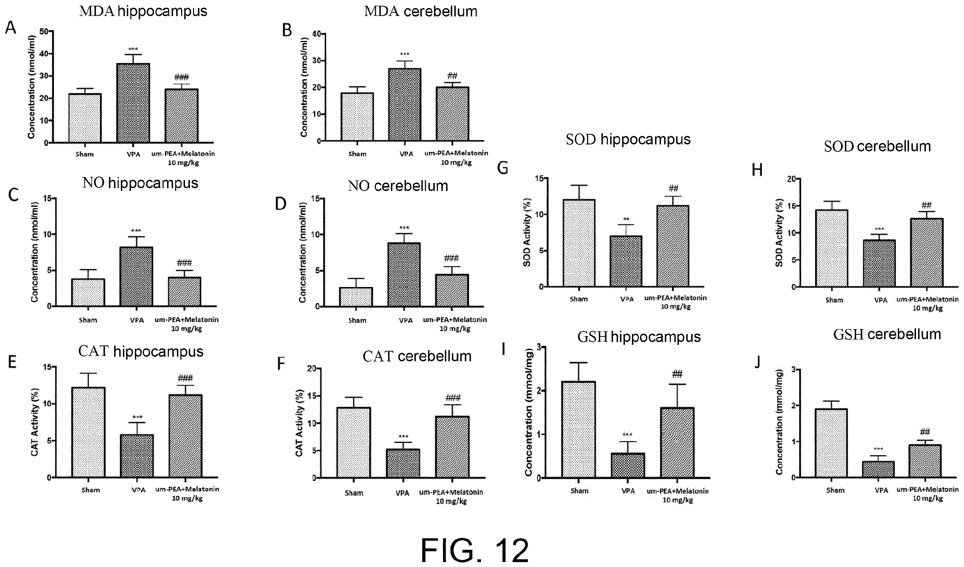

Figure 12 shows graphs related to the ability of the inventive composition to counteract the cerebellar and hippocampal oxidative stress induced by VPA; a significant reduction is noted in lipid peroxidation measured as levels of malondialdehyde (MDA) (Histograms A, B) and nitrogen monoxide (NO) (Histograms C, D) and an increase in the activity of the catalase antioxidant enzymes (CAT) (Histograms E and F) and superoxide dismutase (SOD) (Histograms G and H), as well as the levels of reduced glutathione (GSH) (Histograms I and J) (**p < 0.01 vs. Sham; ***p < 0.001 vs. Sham; ##p < 0.01 vs. VPA; ###p < 0.001 vs. VPA);

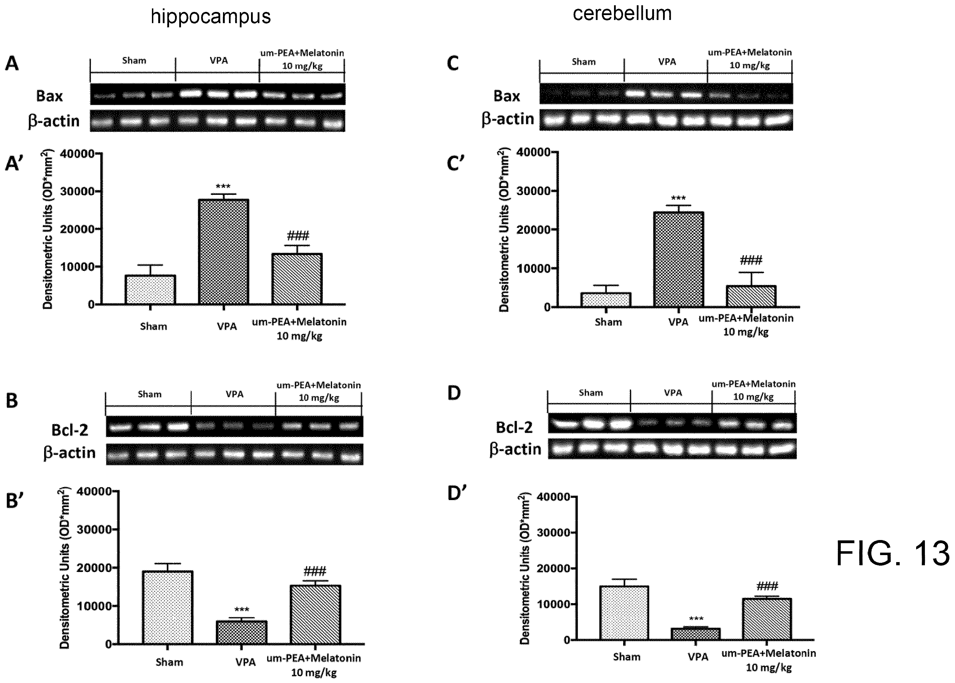

Figure 13 shows graphs related to the contrast by means of the composition of the invention of apoptosis cell death at the cerebellum and hippocampus level, as demonstrated by the reduced expression of the pro-apoptotic factor Bax and increased expression of the anti-apoptotic factor Bcl-2 (***p < 0.001 vs. Sham; ###p < 0.001 vs. VPA);

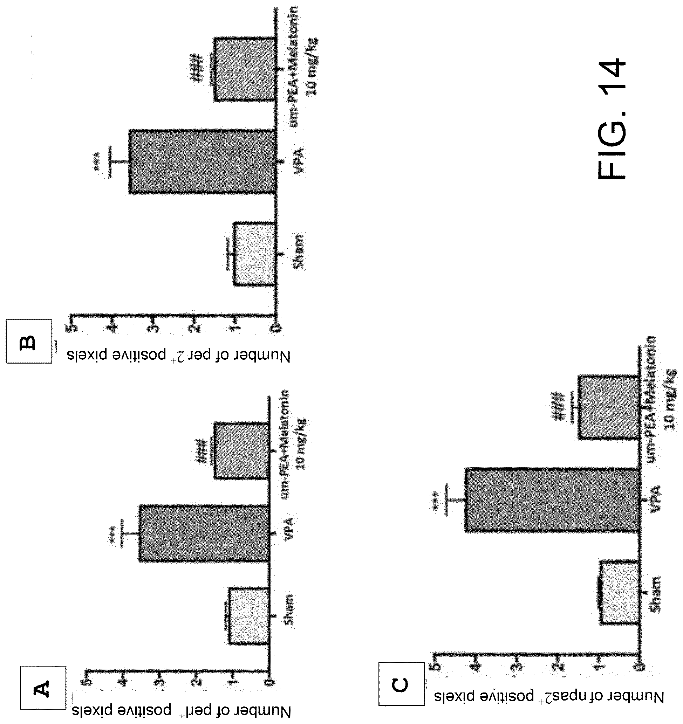

Figure 14 shows graphs related to the contrast of the VPA-induced cerebellar augmentation of per1, per2 and npas2 expression by means of the composition of the invention (***p < 0.001 vs. Sham; ###p < 0.001 vs. VPA);

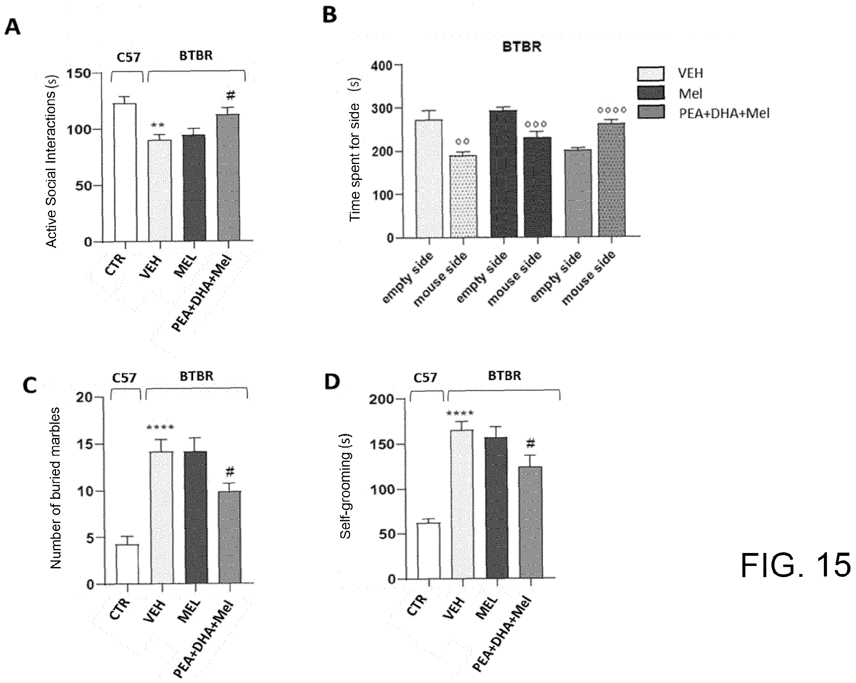

Figure 15 shows graphs relating to the effect of the composition of the invention on the neurobehavioral alterations expressed in BTBR mice compared to control mice (C57), and more particularly on sociability measured with the social interaction test (A) and 3-chamber social interaction test (B), obsessive-compulsive and stereotyped-repetitive behaviors, measured respectively with the Marble Burying test (C) and the Self Grooming test (D). **p < 0.01 vs. C57 VEH; ****p < 0.0001 vs. C57 VEH; #p < 0.05 vs. BTBR VEH; °°p < 0.01 vs. empty side; °°°p < 0.001 vs. empty side; °°°°p < 0.0001 vs. empty side;

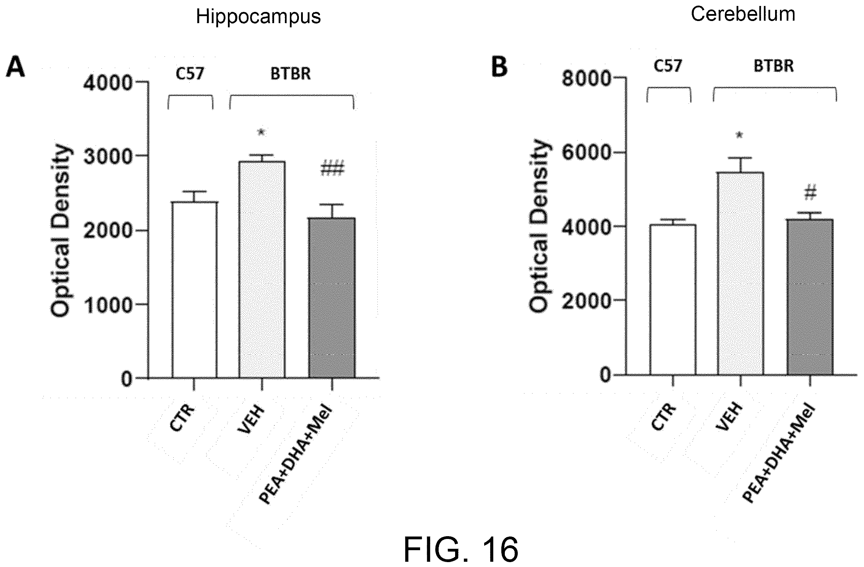

Figure 16 shows graphs relating to the contrast of the increase in hippocampal (A) and cerebellar (B) oxidative stress by the composition of the invention, measured as MDA levels in mice with neurobehavioral alterations (BTBR) compared to control mice (C57). *p < 0.05 vs. CTR; #p < 0.05 vs. VEH; ##p < 0.01 vs. VEH;

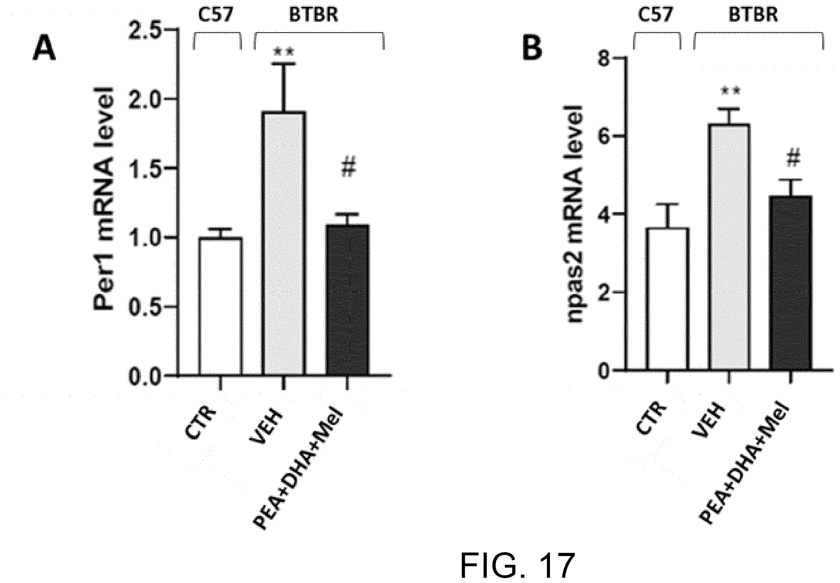

Figure 17 shows graphs relating to the contrast of the cerebellar increase of perl and npas2 expression in BTBR mice versus controls (C57) by the composition of the invention. **p < 0.01 vs. CTR; ^{#}p < 0.05 vs. VEH.

### Detailed description of the invention

The present invention relates in a first aspect to PEA for use in the treatment of ASD, where PEA is administered in association with melatonin or natural precursors thereof, where said administration is separate, combined, or simultaneous.

The term "in association" means both a combination therapy and a therapy in which PEA and melatonin or natural precursors thereof are contained in a single dosage form.

"Separate" administration means an administration of PEA and melatonin or natural precursors thereof in separate dosage forms, administered at different times ranging from 1 minute to several hours, for example 8, 12 or 14 hours apart.

"Combined" administration means an administration of PEA and melatonin or natural precursors thereof contained in a single dosage form, i.e., a pharmaceutical or veterinary composition or formulation, supplement, dietary composition or food for special medical purposes.

"Simultaneous" administration means an administration of PEA and melatonin or natural precursors thereof in separate dosage forms, but administered simultaneously, i.e., within a separation time between the PEA and melatonin administration, or vice versa, not exceeding 1 minute.

PEA can be administered in any form, for example in a non-micronized form, in a micronized form or in an ultra-micronized form.

The term "palmitoylethanolamide (or PEA) in a non-micronized form" means PEA having a particle size distribution, defined as a percentage by volume and measured with the laser light scattering method, represented by a distribution curve having the mode above 10 microns, preferably above 20 microns.

The term "palmitoylethanolamide (or PEA) in a micronized form" means PEA having a particle size distribution, defined as a percentage by volume and measured with the laser light scattering method, represented by a distribution curve having the mode between 6 microns and 10 microns.

The term "palmitoylethanolamide (or PEA) in an ultra-micronized form" means PEA having a particle size distribution, defined as a percentage by volume and measured with the laser light scattering method, represented by a distribution curve having the mode below 6 microns and above 0.5 microns.

Preferably, the PEA is in an ultra-micronized form.

In an embodiment, the PEA in an ultra-micronized form has a particle size distribution as defined above, measured with a Malvern Mastersizer 3000 instrument with Fraunhofer calculation algorithm, where at least 95% by volume, more preferably at least 99% by volume, of particles has a particle size of less than 6 microns.

In a particularly preferred embodiment, the PEA in an ultra-micronized form has a particle size distribution as defined above, measured with a Malvern Mastersizer 3000 instrument with Fraunhofer calculation algorithm, having a mode between 2 and 4 microns and having 100% by volume of particles smaller than 10 microns and at least 60% by volume of particles smaller than 3 microns.

The micronization can be carried out in a fluid jet system (for example, Jetmill^{®} model system) which operates with spiral technology with a compressed air or nitrogen jet capable of exploiting kinetic energy - instead of mechanical energy - to crush the particles. Such apparatuses are conventional and will therefore not be further described, except in relation to the following features:
- Internal diameter of the micronization chamber about 300 mm;
- Fluid jet pressure 10-12 bar;
- Product supply 9-12 kg/h.

Melatonin has the following structural formula:

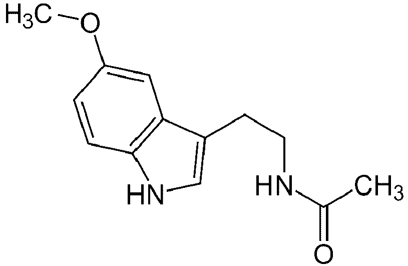

Melatonin (chemically N-acetyl-5-methoxy tryptamine) is a hormone produced by the pineal gland (or epiphysis), the gland at the base of the brain. It acts on the hypothalamus and has the function of regulating the sleep-wake cycle. In addition to humans, it is also produced by other species of animals, plants (phytomelatonin) and microorganisms. Low-dose melatonin is used regularly in sleep-wake cycle disorder therapy.

The term "natural precursors" of melatonin means tryptophan (TRP) and 5-hydroxytryptophan (5H-TRP), which generate serotonin which in turn is converted into melatonin.

The biosynthetic diagram from TRP to 5H-TRP and serotonin is as follows:

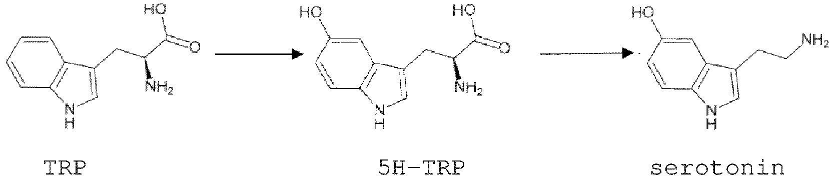

The transformation of TRP into 5H-TRP is carried out by tryptophan hydroxylase, while the subsequent transformation of 5H-TRP into serotonin occurs by means of the aromatic L-amino acid decarboxylase.

Melatonin is finally synthesized by serotonin via N-acyltransferase and acetylserotonin O-methyltransferase.

A further object of the present invention is a composition comprising palmitoylethanolamide and melatonin or natural precursors thereof as defined above. Preferably, the composition of the invention consists of a dry mixture of PEA/melatonin and pharmaceutically acceptable excipients. More preferably, PEA is in micronized (m-PEA) or ultra-micronized (um-PEA) form, still more preferably the palmitoylethanolamide is um-PEA.

Whether administered separately or combined in a single formulation, the PEA and melatonin are administered in a weight ratio between at least 20:1 and 5:1, preferably between at least 12:1 and 8:1. More in particular, when the PEA is in ultra-micronized form, the weight ratio PEA/melatonin will preferably be between at least 11:1 and 8:1, more preferably between at least 10:1 and 9:1. When the PEA is in micronized or non-micronized form, the weight ratio PEA/melatonin will preferably be between at least 20:1 and 10:1, more preferably between at least 18:1 and 12:1.

Based on such weight ratios for which a significant synergistic effect has been highlighted and considering that the dose of melatonin normally considered safe in a subject is between 0.5 and 6 mg/day, the minimum daily dose of PEA, both in a combination therapy and in a PEA/melatonin composition, will be at least between 2.5 mg/day and 120 mg/day. Preferably, in the case of using um-PEA, the minimum daily dose of um-PEA will be between 4 mg/day and 66 mg/day, while in the case of using non-micronized PEA or m-PEA the minimum daily dose will be between 5 mg/day and 120 mg/day.

Such doses may vary depending on the subject and in particular if the subject is of child, adult or elderly age. In fact, in such cases, the daily doses of melatonin considered safe are 1-3 mg/day in children, 0.5-5 mg/day in adults and 0.1-2 mg/day in the elderly, whereby the minimum daily doses of PEA must be calculated accordingly.

When the precursors TRP and 5H-TRP are used in place of melatonin, the weight ratio PEA/TRP or PEA/5H-TRP will preferably be between at least 5:1 and 1:10, more preferably between at least 3:1 and 1:6.

The daily dose of TRP or 5H-TRP is preferably between 30 and 500 mg/day, whereby it will also be possible to calculate the minimum daily dose of PEA based on the weight ratios described above.

Given that, as is widely known from the literature, PEA has an anti-inflammatory activity in general and counteracts neuroinflammation both centrally and peripherally and that such a molecule has a low toxicity, it will be possible to use higher doses of PEA than those described above, which are sufficient to obtain a synergistic effect on autism spectrum disorders.

The additional PEA with respect to the amount of synergistic PEA with melatonin or the natural precursors thereof can also be present in a different form from that used in association with melatonin, TRP or 5H-TRP. For example, if the latter is in the form of um-PEA, the additional PEA may be either um-PEA, m-PEA or non-micronized PEA, or vice versa.

Therefore, the overall daily dose of PEA administered to a subject, either in the form of combination therapy or in the aforesaid composition with melatonin or the natural precursors thereof, can be between 200 and 1500 mg/day, preferably between 400 and 1200 mg/day.

Such daily doses can be divided into dose units for an administration, for example, from 1 to 4 times a day. The dose will also depend on the route chosen for administration. It should be considered that it may be necessary to continuously vary the dosage depending on the age and weight of the patient and also on the severity of the clinical condition to be treated. The exact dose and route of administration will ultimately be at the discretion of the attending physician.

In certain embodiments, the invention relates to the association of PEA, melatonin or natural precursors thereof and docosahexaenoic acid (DHA) or oils suitably titrated in DHA, where the PEA, melatonin and the natural precursors thereof are as defined above.

DHA, also called cervonic acid, is an omega-3 or PUFA n-3 fatty acid. Oceanic cold-water fish are rich in DHA. Most of the DHA present in fish and complex organisms, which live in cold ocean waters, comes from photosynthetic algae. DHA is also commercially produced by microalgae, *Crypthecodinium cohnii* which is a microorganism of the genus *Schizochytrium.* DHA produced using microalgae is of plant origin.

Also in this case a separate, combined or simultaneous administration can be made available and a composition comprising a ternary dry mixture of PEA, melatonin or natural precursors thereof and DHA can be made available, where the weight ratios PEA/melatonin, PEA/TRP and PEA/5H-TRP are as defined above and the weight ratio PEA/DHA is between 1:7 and 1:1, preferably between 1:5 and 1:2, when the PEA is um-PEA, and between 1:1 and 7:1, more preferably between 2:1 and 5:1 when the PEA is non-micronized PEA or m-PEA.

Preferably, the dosages of DHA administered to a patient of child or adolescent age are 700 mg/day or less, or more preferably 500 mg/day or less.

For the purposes of the invention, PEA alone, melatonin or the natural precursors thereof alone or the composition containing PEA and melatonin or natural precursors thereof, optionally in association with DHA, can be included in pharmaceutical or veterinary formulations and can be formulated in dosage forms for oral, buccal, parenteral, rectal, topical or transdermal administration.

For oral administration, the compounds of the invention can be found, for example, in the form of tablets or capsules, hard or soft, prepared in the conventional fashion with pharmaceutically acceptable excipients such as binders (e.g., pregelatinized cornstarch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or inhibiting agents (e.g., sodium lauryl sulfate). The tablets can be coated by methods well known in the art. The liquid preparations for oral administration can be, for example, in the form of solutions, syrups or suspensions or they can be freezedried or granulated products to be reconstituted, before use, with water or other suitable vehicles. Such liquid preparations can be prepared through conventional methods with pharmaceutically acceptable additives such as suspending agents (e.g., sorbitol syrup, cellulose derivatives, or edible hydrogenated fats); emulsifiers (e.g., lecithin or acacia); non-aqueous vehicles (e.g., almond oil, oily esters, ethyl alcohol or fractionated vegetable oils); and preservatives (e.g., methyl- or propyl-p-hydroxybenzoates, sorbic acid, benzoic acid or salts thereof). The preparation can also conveniently contain flavorings, dyes, and sweeteners.

The preparations for oral administration can be appropriately formulated to allow the controlled release of the active constituent.

For buccal administration, the compounds of the invention can be in the form of tablets or pills formulated in the conventional manner, which are suitable for an absorption at the level of the buccal mucosa. Typical buccal formulations are tablets for sublingual administration.

The compounds of the invention can be formulated for parenteral administration by injection. The injection formulations can be presented as a single dose, for example in vials, with an added preservative. The compositions can appear in such a form as suspensions, solutions, or emulsions in oily or aqueous vehicles and can contain agents of the formulation such as suspension, stabilizers and/or dispersants. Alternatively, the active ingredient or the mixture of active ingredients can be found in the form of a powder to be reconstituted, before use, with a suitable vehicle, for example with sterile water.

The compounds of the invention can also be formulated according to rectal formulations such as suppositories or retention enemas, for example containing the basic components of the common suppositories such as cocoa butter or other glycerides.

In addition to the formulations described above, the compounds of the invention can also be formulated as a deposit preparation. Such long-acting formulations can be administered by implantation (e.g., subcutaneously, transcutaneously or intramuscularly) or intramuscular injection. Therefore, for example, the composition can be formulated with appropriate polymer or hydrophobic materials (for example in the form of an emulsion in a suitable oil) or ion exchange resins or as minimally soluble derivatives.

The compounds or composition of the invention can also be administered in the form of oral sprays or nasal sprays.

The invention further relates to dietary compositions, food supplements, complementary feed and foods for special medical purposes (FSMPs) comprising PEA, preferably ultra-micronized PEA, and melatonin for use in the treatment of ASD. In such cases, the daily dose of melatonin must not exceed 1 mg/day.

The term "food for special medical purposes" means products authorized according to regulation (EU) 2016/128. Such a term refers to a product to be administered under medical supervision, thus assimilating such an FSMP to a drug.

The formulations according to the invention can be prepared according to conventional methods, such as those described in Remington's Pharmaceutical Sciences Handbook, Mack Pub. Co., N.Y., USA, 17th edition, 1985 o in Remington, The Science and Practice of Pharmacy, Edited by Allen, Loyd V., Jr, 22nd edition, 2012 or in subsequent editions.

### EXPERIMENTAL SECTION

### Micronization procedure

The PEA was micronized as previously described.

The ultra-micronization was carried out in a fluid jet system (in particular, the Jetmill^{®} model system) which operates with compressed air jet "spiral technology".

Optimal micronization conditions:
- internal diameter of the micronization chamber 300 mm;
- fluid jet pressure 8 bar;
- product supply 9-12 kg/h.

### Determination of the particle size distribution

The determination of the particle size distribution was carried out on a wet sample, after 1-minute sonication.

A Malvern Mastersizer 3000 instrument operating with the LALLS (Low Angle Laser Light Scattering) technique and a Fraunhofer calculation algorithm was used.

The particle size distribution graph is shown in figure 1.

### Biological experimentation

### a. Mixture of PEA-um and melatonin in VPA mice

To evaluate the synergy of a combination PEA-um and melatonin, experimental tests were carried out by oral administration (gastric tube) of a mixture of um-PEA and melatonin (approximately 10:1 ratio) at a dose of 10 mg/kg for two weeks in mice in which an autistic phenotype was induced by a single injection of valproic acid in the form of sodium valproate (VPA).

Furthermore, experimental tests were carried out to determine the synergy of the PEA-Melatonin-DHA combination administered orally to genetically modified BTBR T+tf/J (BTBR) mice, an animal model of neurobehavioral disorders of the ASD type.

Healthy male C57/BL6 mice fed *ad libitum* and housed in cages with controlled sleep/wake cycles were used in the first experimentation. Before the start of the experimentation, the animals were subjected to an acclimatization period of 1 week considering all the experimental procedures and protocols, compliant with the principles of care and welfare of laboratory animals approved by the Italian Ministry of Health (Italian Legislative Decree 2014/26) and European directives (EU Directive 2010/63) and by the ARRIVE guidelines.

To induce the simil-autistic phenotype, 14-day-old animals were chemically treated with a single dose of subcutaneous (s.c.) VPA at a concentration of 400 mg/kg in 100 µl of saline.

The animals were randomized into 6 groups of 20 mice each and treated *per os,* starting from the 15th day of life, with 1.5% carboxymethyl cellulose (CMC) (vehicle used to suspend the molecules), with Melatonin (1 and 10 mg/kg), um-PEA (9 mg/kg) and um-PEA/Melatonin (10 mg/kg in 10:1 ratio, previously mixed dry) daily for 14 days:
Group 1: healthy mice not administered VPA s.c. and treated with CMC 1.5%, Melatonin (1 and 10 mg/kg), um-PEA (9 mg/kg) and um-PEA/Melatonin 10 mg/kg (in 10:1 ratio, previously mixed dry). Since no significant difference was detected between the various Sham groups, only the Sham + vehicle group was considered in the analyses (Sham);
Group 2: mice administered VPA s.c. and treated with 1.5% CMC (VPA);
Group 3: mice administered VPA s.c. and treated with Melatonin (1 mg/kg) suspended in 1.5% CMC (Melatonin 1 mg/kg);
Group 4: mice administered VPA s.c. and treated with Melatonin (10 mg/kg) suspended in 1.5% CMC (Melatonin 10 mg/kg);
Group 5: mice administered VPA s.c. and treated with um-PEA (9 mg/kg) suspended in CMC 1.5% (um-PEA 9 mg/kg);
Group 6: mice administered VPA s.c. and treated with um-PEA/Melatonin (10 mg/kg, in 10:1 ratio, previously mixed dry) suspended in 1.5% CMC (um-PEA+Melatonin 10 mg/kg).

### b. Mixture of PEA-um, melatonin and DHA in BTBR mice

To determine the synergy of a PEA-um, melatonin and DHA combination, a second experimentation was performed, using a different animal model useful for the study of neurobehavioral disorders, and more precisely BTBR mice. These animals are genetically lacking the corpus callosum and have severely reduced hippocampal commissure. Over a period of time comparable to infant infancy, the animals develop behavioral deficits, reduced social interactions, altered play expressions, reduced exploratory behavior, unusual vocalizations, and anxiety.

The mice were randomized into 4 groups of 8 animals each (3 BTBR groups and 1 group of healthy C57 mice used as controls), and treated orally every day for 10 consecutive days, starting from the fourth month of life, according to the following groups of treatment:
I. C57 mice treated with 1.5% carboxymethylcellulose, CMC (CTR);
II. BTBR mice treated with 1.5% CMC (VEH);
III. BTBR mice treated with melatonin 0.1 mg/kg (Mel) resuspended in 1.5% CMC;
IV. BTBR mice treated with the combination of PEA-um 1 mg/kg, DHA 5 mg/kg and melatonin 0.1 mg/kg (PEA+DHA+Mel) resuspended in 1.5% CMC.

The animals underwent the following behavioral tests:
- The social interaction test, also known as a 3-chamber social test (figure 2 and figure 15 A,B) to evaluate i) the interaction time of the animals with an unknown mouse and ii) the sociability index (ratio between the time spent by the mouse exploring the area without and with the new companion). Such a test was performed as described in Crawley, J.N. Designing mouse behavioral tasks relevant to autistic-like behaviors. Ment Retard Dev Disabil Res Rev 2004, 10, 248-258, doi:10.1002/mrdd.20039. In short, the mice were placed inside an arena formed by three chambers communicating with each other through central openings for 5 minutes. During this period, the distance (cm) and the time (s) spent in the different chambers were estimated, so as to evaluate the locomotor activity and the possible preference for one of the arms of the apparatus. After this adaptation period, the animals were confined in the central chamber, while an unknown mouse, inserted in a small metal cage, was placed in one of the two outer chambers. An identical empty metal cage was placed in the remaining free chamber. In this phase of the test, parameters such as the time spent by the animal exploring i) the chambers and ii) the unfamiliar mouse were detected.
- The Novel Object Recognition Test or NOR test (figure 3) to examine cognition and recognition memory by calculating the time spent by the animal exploring new objects with respect to familiar ones. Such a test was performed as described in Lindsay M Lueptow , Novel Object Recognition Test for the Investigation of Learning and Memory in Mice J Vis Exp. 2017 Aug 30; (126) :55718*.* Briefly, on the day before the test, each mouse was allowed to explore the arena for 5 minutes. The following day, two tests (T1 and T2) were carried out in a single session, separated from each other by an interval of 90 min. In the familiarization test (T1), each animal was placed in the arena with two identical objects placed on opposite sides of the apparatus. The familiarization session lasted until the animal explored both objects. The exploration time was counted from the moment the mice directly looked at and smelled the object at a distance of less than 2cm. After the 90 min interval, the object recognition test (T2) was carried out: each animal was relocated in the arena where one of the familiar objects was replaced with a new object. In this phase the time spent by the animals exploring the familiar object and the new object was acquired.
- The repetitive behaviors (self-grooming test) (figure 4A and figure 15 D) and atypical motor stereotypies (walking in circles and backwards, figure 4B). The first test was performed as described in Pellow S., Chopin P., File S.E., Briley M. (1985), Validation of open:closed arm entries in an elevated plus-maze as a measure of anxiety in the rat, "J. Neurosci. Methods" 14, pp. 149-67. The animals were placed in an empty plastic cage for 20 minutes. After an acclimatization period of 10 minutes, the time spent self-grooming was recorded using a specific software able to analyze the repetitive attitude in washing the head, the body, the genital area, the tail and in licking paws and legs. Analyses of atypical motor stereotypies (walking backwards and in circles) were instead observed and recorded by a specialized laboratory technician.
- The Elevated Plus Maze (EPM) (figure 5) to measure animal anxiety. The instrument consists of a cross-shaped apparatus, with two open arms and two closed arms, raised from the ground by about 40-70 cm. This test exploits the rodent's conflict between an aversion to open spaces and an instinct to explore new environments. The test begins by placing the animal in the center of the labyrinth and leaving it free to explore the apparatus for 5 minutes, during which the session is videotaped by means of a camera placed above the apparatus. The behavior of the animals is analyzed through the use of dedicated software, by virtue of which it is possible to quantify the time spent in the four arms and in the central platform. Behaviors indicative of the animal's anxiety status are latency, frequency, and duration of the visits in the open and closed arms. The shorter the time spent in the open arms and on the central platform, the greater the animal's anxiety.
- Marble Burying to study obsessive/compulsive behavior (figure 15C). Briefly, 20 marbles were placed on a grid inside a Plexiglas cage filled with 5 cm of clean litter. Each mouse was placed in the cage and, after a 15-minute session, gently removed to count the number of buried marbles. Since the excessive repetition of behaviors, such as digging, is comparable to the expression of a neophobic and/or compulsive-like behavioral phenotype, the greater the number of buried marbles, the more severe the obsessive-compulsive neurobehavioral alteration;
- The Thermal Nociception test (figure 6) to measure sensitivity to pain. The test allows measuring the time taken by animals to show a reaction to a thermal stimulus (retraction of the paw from a hot surface). In particular, the animal is placed inside a chamber formed by transparent walls, open on the top, the floor of which corresponds to a thermostated hot plate, with temperature maintained at 51.5°C ± 1°C. The perception of the painful stimulus is represented by the attempt to remove the paw from the heat source. The pain is measured as the time elapsed from the instant the animal is placed on the plate to the instant when a behavior indicative of pain perception occurs (the animal jumps or licks its paw) (Hargreaves K, Dubner R, Brown F, Flores C, Joris, A new and sensitive method for measuring thermal nociception in cutaneous hyperalgesia, J. Pain. 1988 Jan;32(1):77-88; doi : 10.1016/0304-3959(88)90026-7)*.*

At the end of the behavioral tests and treatments, the animals were sacrificed for brain sampling, immediately fixed in formalin and then cut into sagittal sections (7 um) stained with hematoxylin and eosin (E/E).

Changes were studied in the density in the Purkinje cell layer (pcs) and neuronal damage in the CA1 and CA3 regions of the hippocampus (figures 7-8) by virtue of the attribution of a score between 0 and 3 (0= normal, 1 = some damaged neurons (<30%), 2=many damaged neurons (30 to 70%) and 3=majority of damaged neurons (> 70%)). All the histological studies were performed blindly.

The protein analyses (Western Blotting) were performed on hippocampal and cerebellar tissues using the primary antibodies anti-GFAP (1:500, Santa Cruz Biotechnology), anti-Iba1 (1:500, Santa Cruz Biotechnology), anti-IkB-α (1:500, Santa Cruz Biotechnology), anti-NF-kB (1:500, Santa Cruz Biotechnology), anti-Bax (1:1000, Santa Cruz Biotechnology), anti-Bcl-2 (1:1000, Santa Cruz Biotechnology) and anti-β-actin (1:5000, Santa Cruz Biotechnology). Protein expression was quantified by densitometry (BIORAD ChemiDoc^{™} XRS+software) and normalized to β-actin expression levels.

Immunohistochemical (IHC) analyses were performed at the level of the cerebellum in tissues derived from VPA animals to assess the number of positive cells for the primary antibodies anti-Period (per)-1, anti-per2 and anti-npas2, proteins involved in homeostatic regulation of sleep, circadian rhythm generation, and memory consolidation.

Due to the structural alterations present in the brain of BTBR animals, it was not possible to perform immunohistochemical analyses to evaluate the number of cells positive for the primary anti-Period (per)-1 and anti-npas2 antibodies. For this reason, Real Time (RT)-PCR analyses were performed to quantify and evaluate the gene expression of the aforementioned sleep genes, at the level of the cerebellum.

Briefly, a small portion of the cerebellum was preserved in RNALater and used for RNA extraction (Trizol, Invitrogen). The extracted RNA was treated with RNAsefree DNAse I (New England Biolabs, Ipswich, MA, USA) and 1ug of total RNA of each sample was reverse transcribed using the RevertAid First Strand cDNA Synthesis Kit (Invitrogen). Complementary DNA (cDNA) was then used as a template to evaluate messenger RNA (mRNA) expression of the perl and npas2 genes. All samples were tested in triplicate and b-actin was used as the housekeeping gene

Further analyses were conducted on hippocampal and cerebellar tissues to assess:
- lipid peroxidation measured as malondialdehyde (MDA) levels (Sigma, Italy). The absorbance of the supernatant was measured with the spectrophotometer at 532 nm (Impellizzeri D et al., Biochem Pharmacol 2016; 119: 27-41);
- nitrogen monoxide (NO) by virtue of the Griess-Ilosvoy reagent (Sigma, Italy) and measured with spectrophotometric technique at 540 nm (Tracey WR et al., J Pharmacol Exp Ther 1995; 272: 1011-1015);
- the activity of the catalase antioxidant enzymes (CAT) (Sinha AK., Anal Biochem 1972; 47: 389-394) and superoxide dismutase (SOD) (Impellizzeri D et al., FASEB J 2019; 33: 11364-1138);
- reduced glutathione (GSH) levels dosed with the 412 nm microplate reader (Ellman GL., Arch Biochem Biophys 1959; 82: 70-77).

Finally, sagittal sections of the brain derived from VPA mice were stained with luxol fast blue (LFB) technique (Ghasemi-Kasman M et al. In vivo conversion of astrocytes to myelinating cells by miR-302/367 and valproate to enhance myelin repair. J Tissue Eng Regen Med 2018; 12:e462-e472) to quantify myelin sheath demyelination (figure 9).

### Statistical analysis

All the values reported in the results were expressed as mean ± standard error of the mean (SEM) of N observations (N = number of animals). Between-group statistical differences for the VPA mice experiments in behavioral score and molecular analyses were analyzed by ANOVA followed by Bonferroni's post-hoc test for multiple comparisons. For experiments conducted in BTBR mice, ANOVA analysis was used followed by Tukey's multiple comparison test.

The p-value < 0.05 was considered significant.

### Results of the experiments

### a. Mixture of PEA-um and melatonin in VPA mice

From the experiments described above and as shown by the results reported in the graphs of figures 2-14, the PEA/melatonin association (in particular in the form of a dry mixture administered as a suspension/solution in the carrier) is capable of:
1. significantly increasing the interaction time with another mouse (indicated as "stranger" in histogram A) of figure 2 more than um-PEA alone (9 mg/kg) and melatonin (10 mg/kg);
2. increasing the sociability index (ratio between the time spent by the mouse exploring the area without and with the new companion), significantly reduced in the VPA mice, more than um-PEA alone (9 mg/kg) and melatonin (10 mg/kg): histogram (B) of figure 2;
3. increasing the time for exploring a new object with respect to a familiar object (indicated as "stranger" and "object" in histogram A in figure 3, respectively) more than um-PEA alone (9 mg/kg) and melatonin (10 mg/kg);
4. increasing the total exploration time of objects more than um-PEA alone (9 mg/kg) and melatonin (10 mg/kg): histogram (B) of figure 3;
5. significantly reducing stereotyped repetitive behaviors more than um-PEA alone (9 mg/kg) and melatonin (10 mg/kg); the behaviors analyzed are self-grooming (histogram A of figure 4) and unnatural repetitive movements such as turning in a circle and walking backwards (histogram B of figure 4);
6. reducing anxiety in autistic animals more than um-PEA alone (9 mg/kg) and melatonin (10 mg/kg), as demonstrated by increased time spent in the open arm of the elevated plus maze (EPM), figure 5;
7. correcting pain sensitivity (hot plate) reduced by VPA (similar to what is found in some autistic children). Once again, the treatment with the mixture under study decreases VPA-induced alteration more than um-PEA alone (9 mg/kg) and melatonin (10 mg/kg), figure 6. It is also interesting to note that melatonin at 1 mg/kg (shown to be inactive in all of the above assessments) in this case worsens rather than improves VPA-induced pain insensitivity. The worsening is significant;
8. reducing the signs of pyramidal cell neurodegeneration in the CA1 and CA3 regions of the hippocampus (these areas are involved in cognitive processes, such as consolidation of memory and spatial memory, orientation, learning new information and movements), reorganizing the architectural structure of the hippocampus itself. The effect is greater than um-PEA alone (9 mg/kg) and melatonin (10 mg/kg), figure 7;
9. increasing, more than um-PEA alone (9 mg/kg) and melatonin (10 mg/kg), the density of the Purkinje cells in the cerebellum (Purkinje cells are essential for the body's motor function. Disorders involving Purkinje cells usually adversely affect patient movement), reduced following VPA injection (figure 8);
10. significantly counteracting the demyelination of the myelin sheath more than um-PEA alone (9 mg/kg) and melatonin (10 mg/kg), figure 9;
11. reducing VPA-induced astrogliosis and microgliosis in the hippocampus and cerebellum, as demonstrated by the lower expression of the specific markers GFAP and Iba-1, respectively (figure 10);
12. counteracting the effects of VPA on nuclear proinflammatory factor (NF-kB) and the "inhibitor" thereof (IkB-α) at the cerebellum and hippocampus level (figure 11). In particular, the mixture raises the levels of IkB-α and reduces those of NF-kB;
13. counteracting the cerebellar and hippocampal oxidative stress induced by VPA (figure 12). In particular, the mixture significantly reduces lipid peroxidation measured as levels of malondialdehyde (MDA) (Histograms A, B) and nitrogen monoxide (NO) (Histograms C, D), while increasing the activity of the catalase antioxidant enzymes (CAT) (Histograms E, F) and superoxide dismutase (SOD) (Histograms G, H), as well as the levels of reduced glutathione (GSH) (Histograms I, J) ;
14. counteracting cell death by apoptosis at the cerebellum and hippocampus level, as demonstrated by the reduced expression of the pro-apoptotic factor Bax and increased expression of the anti-apoptotic factor Bcl-2 (figure 13);
15. counteracting the VPA-induced cerebellar increase in the expression of per1, per2 and npas2 ("clock proteins" the increase of which in transcriptional activity during sleep deprivation leads to an energy deficit in prolonged wakefulness), figure 14.

In the above study, conducted in a VPA injection model of autism, melatonin at 1 mg/kg was never shown to be active, but actually worsened pain insensitivity.

Based on the conversion of "mouse dose" to "human dose" (Reagan-Shaw S, et al., FASEB J. 2008; Nair AB, Jacob S., J Basic Clin Pharm 2016; 7: 27-31; FDA Guidelines), the 1 mg/kg dose of melatonin used in the mixture of the present study corresponds to an equivalent dose of 0.12 mg/kg in the child. For children weighing 10-20 kg, the daily dose of melatonin to be administered mixed with um-PEA would therefore be equal to 1.2 mg - 2.4 mg, which falls within the daily doses envisaged according to the present invention.

Although at a dose of 10 mg/kg melatonin has been shown to be active in the present study, it should be considered that in a child weighing 10-20 kg, such a dose corresponds to a very high equivalent dose, and equal to 12-24 mg, therefore not usable.

In fact, as mentioned above, in most children with autism the dose of melatonin used is in the range of 1-3 mg/day. In adults, the recommended dose is 0.5-5 mg/day, while for the elderly it is recommended to start with a minimum dose of 0.1 mg/day. Therefore, in general terms, melatonin can be administered in a dose range from 0.1 mg/day to 5 mg/day.

High doses of melatonin can induce in children nightmares, drowsiness, headaches, stomach pain, dizziness and grogginess in the daytime, low blood pressure, nocturnal enuresis, more rarely hyperactivation and reduced sleep. Adults may experience side effects such as vivid dreams or nightmares, headaches, dizziness, daytime sleepiness, short-term feelings of depression, stomach cramps, irritability, decreased libido.

From the present study it is evident that the association between um-PEA and melatonin within the expected dose ranges, therefore at doses of both PEA and melatonin within the allowable and safe doses, is synergistically active in the treatment of autism spectrum disorders.

### b. Mixture of PEA-um, Melatonin and DHA in BTBR mice

From the experiments described above and as shown by the results in figures 15-17, only the PEAum/Melatonin and DHA association is able to:
1. improve the social interaction of animals with neurobehavioral disorder (phenotype BTBR)increasing in a statistically significative way with respect to vehicle the time spent in the company of their own kind (Fig. 15A). BTBR animals treated with 0.1 mg/kg melatonin showed no improvement in the behavioral test (Fig. 15A) by continuing to spend their time in the empty side of the area (Fig. 15B);
2. moderate the obsessive/compulsive behaviors of BTBR animals by significantly reducing the burying of marbles (Fig. 15C);
3. moderate repetitive behaviors, as shown by the ability to significantly reduce versus vehicle the time spent by BTBR mice in self-grooming (Fig. 15D). BTBR animals treated with melatonin 0.1 mg/kg showed no improvement in stereotyped repetitive behaviors (Figs. 15C - 15D);
4. counteract the high levels of oxidative stress both at the hippocampal and cerebellar levels in BTBR mice. In particular, the mixture significantly reduces lipid peroxidation measured as MDA, bringing it back to physiological levels both in the hippocampus and in the cerebellum (Figs. 16A and 16B);
5. Normalize the expression of the genes perl and npas2, involved in the generation of circadian rhythms (central to the regulation of sleep and wakefulness), which were significantly increased in the cerebellum of BTBR mice (Figs. 17A - 17B).

The present invention thus provides a method for treating neurobehavioral disorders, such as e.g. ASD comprising or consisting of administering to a subject, PEA, preferably in ultra-micronized form, and melatonin or natural precursors thereof, and optionally DHA, where said administration is separate, combined (i.e., in a single dosage form) or simultaneous and where at least the melatonin is administered at a dosage at which, when administered alone, it is inactive.

In particular, the disorders which can be treated according to the invention are neurobehavioral disorders accompanied by restlessness, irritability, sleep disorders, and potentially stereotypies and associated with:
A. neurodevelopmental disorders of both humans and pets (dogs and cats), such as autism spectrum disorders (ASD) and attention deficit/hyperactivity disorder (ADHD), including, but not limited to, if in comorbidity with epilepsy (such disorders typically occur in humans, but share common traits with similar neurobehavioral disorders of dogs);
B. anxious/phobic states (e.g., noise phobia, separation anxiety).

It has also been seen that the administration of the compounds or compositions according to the invention is able to treat neuro-behavioral disorders accompanied by restlessness, irritability, sleep disorders and, potentially, stereotypies connected to dementias and senile dementias, also in dog and cat, such as the Cognitive Dysfunction Syndrome.

The invention will now be further described through the following formulation examples.

### Formulation examples

um-PEA = Ultra-micronized palmitoylethanolamide
m-PEA = Micronized palmitoylethanolamide
non-m PEA = non-micronized palmitoylethanolamide

### Example 1 - Soft gelatin capsules

### format 12 squeezable Twist-off

### Capsule content:

| | |
|---|---|
| um-PEA | 150.00 mg |
| melatonin | 3.00 mg |
| Peanut oil | 470.00 mg |
| Soy lecithin | 20.00 mg |
| Alpha-tocopherol | 10.00 mg |
| Glyceryl monostearate | 10.00 mg |

### Composition of the capsule:

| | |
|---|---|
| Bovine gelatin | 237.00 mg |
| glycerol | 130.00 mg |
| Water | 19.00 mg |
| Pigments | 0.07 mg |

### Example 2 - Syrup

### Composition per 100 ml:

| | |
|---|---|
| Sucrose | 25.0 g |
| m-PEA | 12.0 g |
| Melatonin | 100 mg |
| DHA with 55% titer | 5.0 g |
| Microcrystalline cellulose | 1.35 g |
| Natural tocopherol (1000 IU/g) | 1.0 g |
| Sodium Carboxymethyl cellulose | 0.65 g |
| Sorbitan monooleate | 0.40 g |
| Polysorbate 80 | 0.10 g |
| Natural flavoring | 0.10 g |
| Potassium sorbate | 0.09 g |
| Benzoic acid | 0.07 g |
| Citric acid | 0.05 g |
| Water | q.s. to 100 ml. |

### Example 3 - Dispersible granules

### Content of the single-dose sachet:

| | |
|---|---|
| Palmitoylethanolamide | 900 mg |
| Melatonin | 5 mg |
| Maltodextrins | 500 mg |
| Fructose | 300 mg |
| Dextrose | 200 mg |
| Tocopherol acetate 50% (powder on silica) | 200 mg |
| Citric acid | 50 mg |
| Pluronic F-68 | 50 mg |
| Natural flavoring | 50 mg |
| Magnesium Stearate | 10 mg |
| Polysorbate 80 | 10 mg |

### Example 4 - Tablets

### Content of the single-dose tablet:

| | |
|---|---|
| m-PEA | 300 mg |
| Palmitoylethanolamide-um | 10 mg |
| Melatonin | 1 mg |
| Maltodextrins | 60 mg |
| Microcrystalline cellulose | 100 mg |
| Cross-linked sodium carboxymethyl cellulose | 25 mg |
| Polyvinylpyrrolidone | 10 mg |
| Magnesium stearate | 6 mg |
| Colloidal anhydrous silica | 6 mg |
| Polysorbate 80 | 8 mg |
| Coating agent | 30 mg |

### Example 5 - Gastro-protected tablets

### Content of the single-dose tablet:

| | |
|---|---|
| um-PEA | 600 mg |
| Melatonin | 3 mg |
| Microcrystalline cellulose | 150 mg |
| Cross-linked sodium carboxymethyl cellulose | 90 mg |
| Polyvinylpyrrolidone | 40 mg |
| Magnesium stearate | 8 mg |
| Colloidal anhydrous silica | 6 mg |
| Polysorbate 80 | 8 mg |
| Coating agent gastro-resistant | 40 mg |

### Example 6 - Chewable tablets

### Content of the single-dose tablet:

| | |
|---|---|
| m-PEA | 300 mg |
| Melatonin | 1 mg |
| Microcrystalline cellulose | 200 mg |
| Calcium phosphate dihydrate | 95 mg |
| Sorbitol | 80 mg |
| Fructose | 230 mg |
| Citric acid | 25 mg |
| Cross-linked sodium carboxymethyl cellulose | 50 mg |
| Polyvinylpyrrolidone | 40 mg |
| Magnesium stearate | 9 mg |
| Colloidal anhydrous silica | 9 mg |
| Sucrose palmitate | 6 mg |
| Natural flavoring | 10 mg |

### Example 7 - Hard gelatin capsules

### Content of the single-dose tablet:

### Vegetable gelatin capsule (HPMC) format 3

| | |
|---|---|
| um-PEA | 50 mg |
| Melatonin | 1 mg |
| Maltodextrin | 30 mg |
| Microcrystalline cellulose | 50 mg |
| Polyvinylpyrrolidone | 5 mg |
| Magnesium stearate | 4 mg |
| Colloidal anhydrous silica | 4 mg |
| Polysorbate 80 | 2 mg |

### Example 8 - Sublingual tablets

### Content of the single-dose tablet:

| | |
|---|---|
| um-PEA | 10 mg |
| Melatonin | 2 mg |
| Lactose | 30 mg |
| Corn dextrins | 10 mg |
| Polyvinylpyrrolidone | 5 mg |
| Sucrose palmitate | 5 mg |
| Natural flavoring | 3 mg |

### Example 9 - Multi-dose oral spray

### (1 spray corresponds to 100 microliters)

### Content of the 12ml solution:

| | |
|---|---|
| um-PEA | 960 mg |
| Melatonin | 120 mg |
| Sucrose | 600 mg |
| Pluronic F-68 | 600 mg |
| Sucrose palmitate | 150 mg |
| Stevioside | 3 mg |
| Natural flavoring | 20 mg |
| Citric acid | 20 mg |
| Potassium Sorbate | 1 mg |
| Benzoic Acid | 0.8 mg |
| Water | q.s. to 12ml |

### Example 10 - Pediatric suppositories

### Content of the single-dose suppository:

| | |
|---|---|
| Palmitoylethanolamide | 100 mg |
| Melatonin | 1 mg |
| Suppocire BS2X | 850 mg |
| Natural tocopherol | 50 mg |

### Example 11 - Soft gelatin capsules

### Capsule content:

| | |
|---|---|
| um-PEA | 150.00 mg |
| melatonin | 3.00 mg |
| DHA with 55% titer | 435.00 mg |
| Peanut oil | 40.00 mg |
| Soy lecithin | 20.00 mg |
| Alpha-tocopherol | 10.00 mg |
| Glyceryl monostearate | 10.00 mg |

| Composition of the capsule: | |
|---|---|
| Bovine gelatin | 237.00 mg |
| glycerol | 130.00 mg |
| Water | 19.00 mg |
| Pigments | 0.07 mg |

### Example 12 - Single-dose sachet, oral suspension

### Content of the sachet:

| | |
|---|---|
| um-PEA | 900 mg |
| melatonin | 3 mg |
| Corn dextrin | 2500 mg |
| Sodium carboxymethyl cellulose | 135 mg |
| Microcrystalline cellulose | 85 mg |
| Sucrose Palmitate | 10 mg |
| Potassium Sorbate | 9 mg |
| Benzoic acid | 7 mg |
| Natural flavoring | 20 mg |
| Stevioside | 1,5 mg |
| Water | 8050 mg |

### Example 13 - Tablets

### Content of the single-dose tablet:

| | |
|---|---|
| m-PEA | 300 mg |
| Tryptophan | 300 mg |
| Maltodextrins | 120 mg |
| Microcrystalline cellulose | 200 mg |
| Cross-linked sodium carboxymethyl cellulose | 60 mg |
| Polyvinylpyrrolidone | 20 mg |
| Magnesium stearate | 8 mg |
| Colloidal anhydrous silica | 8 mg |
| Polysorbate 80 | 10 mg |
| Coating agent | 40 mg |

### Example 14 - Multi-dose oral suspension

### 100ml bottle

| | |
|---|---|
| um-PEA | 10.00 g |
| Tryptophan | 10.00 g |
| Corn dextrin | 1500 g |
| Sodium carboxymethyl cellulose | 1350 mg |
| Microcrystalline cellulose | 850 mg |
| Sucrose Palmitate | 100 mg |
| Potassium Sorbate | 90 mg |
| Benzoic acid | 70 mg |
| Water | q.s. to 100 ml |

### Example 15 - Gastro-protected tablets

### Content of the single-dose tablet:

| | |
|---|---|
| Palmitoylethanolamide-um | 400 mg |
| 5-Hydroxytryptophan | 200 mg |
| Microcrystalline cellulose | 150 mg |
| Cross-linked sodium carboxymethyl cellulose | 90 mg |
| Polyvinylpyrrolidone | 40 mg |
| Magnesium stearate | 8 mg |
| Colloidal anhydrous silica | 6 mg |
| Polysorbate 80 | 8 mg |
| Gastro-resistant coating agents | 40 mg. |

## Claims

1. Palmitoylethanolamide for use in the treatment of autism spectrum disorders (ASD), wherein the palmitoylethanolamide (PEA) is administered in association with melatonin or triptophan (TRP) or 5-hydroxytriptophan (5H-TRP), wherein said administration is separate, combined or simultaneous.

2. Palmitoylethanolamide for use according to claim 1, wherein the palmitoylethanolamide is:
- in a non-micronized form, having a particle size distribution, defined as a percentage by volume and measured by the laser light scattering method, represented by a distribution curve having the mode above 10 microns, preferably above 20 microns, or
- in a micronized form, having a particle size distribution, defined as a percentage by volume and measured by the laser light scattering method, represented by a distribution curve having the mode between 6 microns and 10 microns, or
- in an ultra-micronized form having a particle size distribution, defined as a percentage by volume and measured by the laser light scattering method, represented by a distribution curve having the mode below 6 microns and above 0.5 microns.

3. Palmitoylethanolamide for use according to claim 2, wherein, when the palmitoylethanolamide is in ultra-micronized form, it has a particle size distribution, defined as a percentage by volume and measured by the laser light scattering method, measured with a Malvern Mastersizer 3000 instrument with Fraunhofer calculation algorithm, wherein at least 95% by volume, preferably at least 99% by volume, of particles has a particle size less than 6 microns, and wherein preferably said ultra-micronized palmitoylethanolamide has a particle size distribution, defined as a percentage by volume and measured by the laser light scattering method, measured with a Malvern Mastersizer 3000 instrument with Fraunhofer calculation algorithm, having a mode between 2 and 4 microns and having 100% by volume of particles less than 10 microns and at least 60% by volume of particles less than 3 microns.

4. Palmitoylethanolamide for use according to any one of claims 1 to 3, wherein PEA and melatonin are administered in a weight ratio between at least 20:1 and 5:1, preferably between at least 12:1 and 8:1.

5. Palmitoylethanolamide for use according to claim 4, wherein, when PEA is in an ultra-micronized form, the PEA/melatonin weight ratio is between at least 11:1 and 8:1, or between at least 10:1 and 9:1 and, when PEA is in a micronized or non-micronized form, the PEA/melatonin weight ratio is between at least 20:1 and 10:1, or between at least 18:1 and 12:1.

6. Palmitoylethanolamide for use according to any one of claims 1 to 5, wherein the minimum daily dose of PEA, in both a combination therapy and a PEA/melatonin composition, is at least between 2.5 mg/day and 120 mg/day, or when PEA is um-PEA, the minimum daily dose of um-PEA is between 4 mg/day and 66 mg/day, or when PEA is non-micronized PEA or m-PEA, the minimum daily dose is between 5 mg/day and 120 mg/day; and wherein the daily dose of melatonin is between 0.5 and 6 mg/day.

7. Palmitoylethanolamide for use according to any one of claims 1 to 3, wherein the PEA/TRP or PEA/5H-TRP weight ratio is between at least 5:1 and 1:10, or between at least 3:1 and 1:6, and wherein the minimum daily dose of PEA, in both a combination therapy and a PEA/TRP or PEA/5H-TRP composition, is at least between 2.5 mg/day and 120 mg/day, or when PEA is um-PEA, the minimum daily dose of um-PEA is between 4 mg/day and 66 mg/day, or when PEA is non-micronized PEA or m-PEA, the minimum daily dose is between 5 mg/day and 120 mg/day; and wherein the daily dose of TRP or 5H-TRP is between 30 and 500 mg/day.

8. Palmitoylethanolamide for use according to any one of claims 1 to 7, in combination with docosahexaenoic acid (DHA) or oils suitably titrated in DHA.

9. Palmitoylethanolamide for use according to claim 8, wherein the PEA/DHA weight ratio is between 1:7 and 1:1, or between 1:5 and 1:2, when PEA is um-PEA, and between 1:1 and 7:1, or between 2:1 and 5:1, when PEA is non-micronized PEA or m-PEA, or wherein the doses of DHA administered to a child or adolescent are equal to or less than 700 mg/day, or equal to or less than 500 mg/day.

10. Palmitoylethanolamide for use according to any one of claims 1 to 9, wherein the overall daily dose of PEA administered to a subject is between 200 and 1500 mg/day, or between 400 and 1200 mg/day and wherein, when PEA is administered in combination or in a composition with melatonin, the daily dose of melatonin administered to a subject is between 0.1 and 5 mg/day.

11. Palmitoylethanolamide for use according to any one of claims 1 to 10, wherein palmitoylethanolamide and melatonin, TRP or 5H-TRP and optionally DHA are contained in pharmaceutical or veterinary formulations and are formulated in dosage forms for oral, buccal, parenteral, rectal, topical, or transdermal administration, or are contained in dietary compositions, food supplements, complementary feeds, or food for special medical purposes (FSMP).

12. Palmitoylethanolamide for use according to any one of claims 1 to 16, wherein the autism spectrum disorders are neurobehavioral disorders accompanied by restlessness, irritability, sleep disorders, and potentially, stereotypies and associated with neurodevelopmental disorders of both humans and pets (dogs and cats), such as autism spectrum disorders (ASD) and attention deficit/hyperactivity disorder (ADHD), including, but not limited to, if in comorbidity with epilepsy.

13. Palmitoylethanolamide for use in the treatment of neuro-behavioral disorders accompanied by restlessness, irritability, sleep disorders and, potentially, stereotypies connected to anxious/phobic states (e.g., noise phobia, separation anxiety), dementias and senile dementias, also in dog and cat, such as the Cognitive Dysfunction Syndrome, in which palmitoylethanolamide is administered in combination with melatonin or with TRP or 5H-TRP, in which said administration is separate, combined or simultaneous.

14. A composition comprising or consisting of a mixture of palmitoylethanolamide, preferably ultramicronized palmitoylethanolamide, melatonin or TRP or 5H-TRP, optionally DHA and pharmaceutically acceptable excipients, wherein the PEA/melatonin weight ratio is preferably between at least 11:1 and 8:1, or between at least 10:1 and 9:1, and when PEA is in a micronized or non-micronized form, the PEA/melatonin weight ratio is preferably between at least 20:1 and 10:1, or between at least 18:1 and 12:1, or wherein the PEA/TRP or PEA/5H-TRP weight ratio is preferably between at least 5:1 and 1:10, or between at least 3:1 and 1:6.

15. A pharmaceutical or veterinary formulation, dietary compositions, food supplements, complementary feeds, or foods for special medical purposes comprising the composition according to claim 14, wherein, preferably, when PEA is associated to melatonin, PEA is between 200 and 1500 mg and melatonin is between 0.1 and 5 mg.
